(19) Europäisches Patentamt
European Patent Office
Office européen des brevets

(11) Veröffentlichungsnummer: **0 072 940**
**B1**

(12) **EUROPÄISCHE PATENTSCHRIFT**

(45) Veröffentlichungstag der Patentschrift: **27.05.87**

(21) Anmeldenummer: **82107055.4**

(22) Anmeldetag: **04.08.82**

(51) Int. Cl.$^4$: **C 07 C 43/166,** C 07 C 69/22, C 07 C 41/01, C 07 C 67/08, C 07 F 9/10

# (54) Zwischenprodukte zur Herstellung von Glycerinderivaten.

(30) Priorität: **04.08.81 DE 3130867**

(43) Veröffentlichungstag der Anmeldung:
**02.03.83 Patentblatt 83/09**

(45) Bekanntmachung des Hinweises auf die Patenterteilung:
**27.05.87 Patentblatt 87/22**

(84) Benannte Vertragsstaaten:
**AT BE CH DE FR GB IT LI LU NL SE**

(56) Entgegenhaltungen:
**EP-A-0 035 375**
**DE-A-2 345 057**
**DE-A-2 745 810**

**Chemical Abstracts, Band 68, Nr. 11, 11. März 1968, Columbus, Ohio, USA, Y.G. MOLOTKOVSKII et al. "Synthesis of (R)-3-(1,2-distearoylglycerylphosphoryl)-1-L-alanylglycerol and study of the configurations of bacterial lipoamino acids, Seite 4862, abstract no. 50024k**

***Die Akte enthält technische Angaben, die nach dem Eingang der Anmeldung eingereicht wurden und die nicht in dieser Patentschrift enthalten sind.***

(73) Patentinhaber: **Max-Planck-Gesellschaft zur Förderung der Wissenschaften e.V.**
**Bunsenstrasse 10**
**D-3400 Göttingen (DE)**

(72) Erfinder: **Eibl, Hansjörg, Prof., Dr.**
**Steinweg 51**
**D-3406 Bovenden (DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

(56) References cited:
**Chemical Abstracts, Band 69, Nr. 7, 12. August 1968, Columbus, Ohio, USA, Y.G. MOLOTKOVSKII et al. "Chemistry of lipids. IX. Synthesis of R-alpha-L-phosphatidyl-y-L-alanyl glycerol and comparison of synthetic O-alanyl esters of phosphatidyl glycerol with natural lipoamino acid from Clostridium welchii", Seite 2600, abstract no. 27753j**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 072 940 B1

(56) References cited:
**Chemical Abstract Band 72, Nr. 11, 16. März 1970, Columbus, Ohio, USA, H. EIBL et al. "Phosphorylation of 1-alkenyl-2-acylglycerol and preparation of 2-acylphosphoglycerides", Seite 344, abstract nr. 54647w**

## Beschreibung

Die Erfindung betrifft Zwischenprodukte zur Herstellung von Glycerinderivaten, wie Glycerinestern und -äthern.

Glycerinester kommen in der Natur als Fette (Triacylglycerine und als Glycerinphosphatide (Diacylglycerinphosphorsäure-Derivate) vor. Sowohl Fette als auch Glycerinphosphatide sind biologisch und technisch äußerst wichtiqe Substanzen. Aufgrund ihrer biologischen und physikalisch-chemischen Eigenschaften finden diese Stoffklassen oder ihre Verarbeitungsprodukte (z.B. die Verseifungsprodukte der Fette) weit verbreitete Anwendung in der Lebensmittelindustrie und in der kosmetischen Industrie, aber auch in vielen anderen Bereichen, wie z.B. als Hilfsmittel in der Leder- und Textilindustrie.

In den natürlich vorkommenden Triacrylglycerinen der Fette oder in den natürlich vorkommenden Glycerinphosphatiden sind meistens zwei bzw. drei verschiedene Fettsäuren enthalten. Darüber hinaus sind die natürlich vorkommenden Fette stets Gemische zahlreicher Triglyceride.

Die Isolierung reiner, einheitlicher Triacylglycerine aus natürlichen Fetten oder von Glycerinphosphatiden aus natürlichen Geweben ist deshalb außerordentlich schwierig und kostspielig. Ihre Synthese ist vor allem dann problematisch, wenn Glycerinester oder Glycerinphosphatide hergestellt werden sollen, in denen Glycerin mit ganz bestimmten Säureesten in ganz bestimmter Stellung acryliert sein soll.

Molotkovski und Bergl'son (Chemical Abstract *69*, 27 753j und Chemical Abstract *68*, 50 024k) beschreiben die Synthese von Phosphorsäurediglycerinestern. Dabei wird die polare Phosphatgruppe durch Umsetzen von Glycerinhalogeniden, die zuvor in einem aufwendigen Syntheseverfahren hergstellt werden müssen, mit Silberphosphaten in das Grundgerüst eingeführt.

E. Cubero Robles et al. (Rec. Trav. Chem. *86* (1976) 762; Biochem. Biophys. Acta *187* (1969) 520) beschreiben z.B. eine Synthese von Glycerinphosphatiden mit gemischten Fett säureresten durch Acylierung von 1-Palmitoyl-sn-glycerin-3-phosphorchlorin und Fettsäureanhydriden in Gegenwart von $Na_2O$. Bei diesem Verfahren findet jedoch in hohem Maße ein Austausch der Acrylgruppen statt, wodurch die Selektivität, d.h. ganz gezielte Einführung bestimmter Acrylreste in bestimmter Stellung stark vermindert wird (vgl. K.M.W. Keough, P.J. Davis, Biochemistry *18* (1979) 1453). Ein weiterer Nachteil dieses Verfahrens ist es, daß während der Acylierungsstufe in erheblichem Maße auch eine Wanderung des Phosphorsäurerests aus der 3- in die 2-Stellung erfolgt. Die Acylierung ist außerdem nur nach vorhergehender Einführung von Aminoschutzgruppen und mit einem großen Überschuß an Säureanhydrid durchführbar, wodurch das Verfahren weiter kompliziert und insbesondere bei Verwendung teurer Fettsäuren, kostspielig wird. Über die Möglichkeiten und die Problematik der Synthese von Glycerinderivaten vgl. auch Keough und Davis, Biochem. *18* (1979) 1453.

Aufgabe der vorliegenden Erfindung ist es deshalb, Ausgangsprodukte und Wege zur Herstellung von Glycerinestern mit Fettsäure- und/oder Phosphorsäureresten, aber auch von entsprechenden Äthern, in denen ein oder mehrere Fettsäurereste durch einen Alkylrest ersetzt sind, bereitzustellen, die die aufgezeigten Nachteile vermeiden und es ermöglichen, Glycerinderivate, und insbesondere Derivate mit verschiedenen Resten, stellungsspezifisch und mit hoher Selektivität auf einfache und wirtschaftliche Weise herzustellen.

Diese Aufgabe wird gelöst durch die Bereitstellung von Zwischenprodukten der Formel II, aus denen Glycerinderivate mit verschiedenen Resten stellungsspezifisch und mit hoher Selektivität hergestellt werden können.

Gegenstand der Erfindung sind deshalb die Glycerinderivate (Zwischenprodukte) der Formel

$$\begin{array}{l} CH_2—O—R_1' \\ | \\ CH—O—R_2' \\ | \\ CH_2—O—R_3', \end{array} \qquad (II)$$

worin jeder der Reste $R_1'$, $R_2'$ und $R_3'$ verschieden ist und eine Benzylgruppe oder Allylgruppe bedeutet, $R_1'$ und $R_3'$ auch eine Tritylgruppe. Von diesen Verbindungen sind bevorzugt das 1-Trityl-2-allyl-3-benzyl-en-glycerin und das 1-Trityl-2-benzyl-3-allyl-sn-glycerin.

Die Glycerinderivate der Formel II werden dadurch hergestellt, daß man in ein 1- (oder 3-)-Benzyl (oder Allyl-)-glycerin in 3- (oder 1-)-Stellung die Tritylgruppe einführt und danach in die 2-Stellung die Allyl (oder Benzyl)-Gruppe. Dieses Verfahren ist ebenfalls Gegenstand der vorliegenden Erfindung.

Es hat sich nunmehr gezeigt, daß man ausgehend von den Zwischenprodukten der Formel II auf einfache und wirtschaftliche Weise Glycerinderivate mit verschiedenen Resten stellungsspezifisch und mit hoher Selektivität herstellen kann. Gegenstand der Erfindung ist daher auch ein Verfahren zur Weiterverarbeitung der Verbindungen der Formel II zur Herstellung von Glycerinderivaten der Formel I

$$\begin{array}{l} CH_2—O—R_1 \\ | \\ CH—O—R_2 \\ | \\ CH_2—O—R_3 \end{array} \qquad (I)$$

worin jeder der Reste $R_1$, $R_2$ und $R_3$ verschieden ist und einen gesättigten Alkylrest R, einen gesättigten Acylrest —COR oder Wasserstoff darstellen, wobei R eine geradkettige oder verzweigte Alkylgruppe mit 1 bis 25 C-Atomen bedeutet, oder einen Arylalkylrest mit 1 bis 25 C-Atomen in der Alkylkette, und worin zwei der Reste auch ungesättigt sein können, und $R_3$ auch die Gruppe

$$\begin{array}{l} P(O)—O—(CH_2)_6—A \\ | \\ O^{\ominus}(Z^{\oplus})_n \end{array}$$

bedeuten kann, worin

A ein Halogenatom, die Gruppe

$$\overset{\oplus}{N}X_1X_2X_3$$

oder auch Wasserstoff ist, $X_1$, $X_2$ und $X_3$ gleich oder verschieden sind und Wasserstoff oder eine geradkettige oder verzweigte Alkylgruppe mit 1 bis 6 C-Atomen, und vorzugsweise Wasserstoff oder Methyl, bedeuten,

y eine ganze Zahl von 2 bis 12, vorzugsweise von 2 bis 6 ist, oder, wenn A ein Wasserstoffatom ist, auch O sein kann,

$Z^{\oplus}$ ein Äquivalent eines ein- oder drei-, und vorzugsweise zweiwertigen Kations ist und

n 1 oder, wenn A die Gruppe

$$\overset{\oplus}{N}X_1X_2X_3$$

bedeutet, O ist,

und worin, wenn A die Gruppe

$$\overset{\oplus}{N}X_1X_2X_3$$

bedeutet, $—(CH_2)_y—$ auch durch eine Carboxylgruppe substituiert sein kann, das dadurch gekennzeichnet ist, daß man von Verbindungen der Formel II ausgeht, und, wenn die Reste $R_1$, $R_2$ und $R_3$ gesättigt sind, oder nur einer der Reste ungesättigt ist,

a) die Tritylgruppe unter schwach sauren Bedingungen abspaltet und die dadurch entstehende OH-Gruppe dann durch Verätherung (für einen von Benzyl verschiedenen Arylalkylrest) oder Acylierung in eine gesättigte Gruppe $OR_1$ oder $OR_3$ überführt oder durch Phosphorilierung in eine Gruppe $OR_3$, danach

b) die Allylgruppe in die Propenylgruppe umlagert und diese abspaltet, und die dadurch entstehende OH-Gruppe, wenn erwünscht, dann durch Verätherung (für einen von Benzyl verschiedenen Arylalkylrest) oder Acylierung in eine gesättigte Gruppe $OR_1$, $OR_2$ oder $OR_3$ oder durch Phosphorilierung in eine Gruppe $OR_3$ überführt, wobei die Umlagerung der Allylgruppe in die Propenylgruppe auch bereits in der Stufe a) vor der Verätherung, Acylierung oder Phosphorilierung erfolgen kann, und anschließend, wenn erwünscht,

c) die Benzylgruppe durch katalytische Hydrogenolyse entfernt und die dadurch entstehende OH-Gruppe, wenn erwünscht, durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, oder, wenn die Reste gesättigt oder ungesättigt sind,

$a_1$) die Tritylgruppe abspaltet und die entstehende OH-Gruppe mit einer Tetrahydropyranylgruppe veräthert, danach

$b_1$) die Alkylgruppe abspaltet und die entstehende OH-Gruppe durch Verätherung (für einen von Benzyl verschiedenen Arylalkylrest) oder Acylierung in eine gesättigte Gruppe $OR_1$, $OR_2$ oder $OR_3$ oder durch Phosphorilierung in eine Gruppe $OR_3$ überführt,

$c_1$) danach die Tetrahydropyranylgruppe oder, wenn erwünscht, die Benzylgruppe abspaltet und die entstehende OH-Gruppe durch Verätherung (für einen von Benzyl verschiedenen Arylalkylrest) oder Acylierung in eine gesättigte (nach Abspaltung der Tetrahydropyranylgruppe) oder gesättigte oder ungesättigte (nach Abspaltung der Benzylgruppe) Gruppe $OR_1$, $OR_2$ oder $OR_3$ oder durch Phosphorilierung in eine Gruppe $OR_3$ überführt und anschließend

$d_1$) die verbleibende Tetrahydropyranylgruppe, oder, wenn erwünscht, Benzylgruppe abspaltet und, wenn erwünscht, die entstehende OH-Gruppe durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, oder

$a_2$) die Tritylgruppe abspaltet und die entstehende OH-Gruppe mit einer langkettigen gesättigten Fettsäure mit 10 bis 25 C-Atomen acyliert, danach

$b_2$) die Allylgruppe abspaltet und die entstehende OH-Gruppe mit einer Tetrahydropyranylgruppe veräthert und

$c_2$) wenn erwünscht, den langkettigen Acylrest abspaltet und die entstehende OH-Gruppe durch

Verätherung (für einen von Benzyl verschiedenen Arylalkylrest) oder Acylierung in eine gesättigte Gruppe $OR_1$ oder $OR_3$ oder durch Phosphorilierung in eine Gruppe $OR_3$ überführt, und danach

$d_2$) die Tetrahydropyranylgruppe oder, wenn erwünscht, die Benzylgruppe abspaltet und die entstehende OH-Gruppe durch Verätherung (für einen von Benzyl verschiedenen Arylalkylrest) oder Acylierung in eine gesättigte (nach Abspaltung der Tetrahydropyranylgruppe) oder gesättigte oder ungesättigte (nach Abspaltung der Benzylgruppe) Gruppe $OR_1$, $OR_2$ oder $OR_3$ oder durch Phosphorilierung in eine Gruppe $OR_3$ überführt und anschließend

$e_2$) die verbleibende Tetrahydropyranylgruppe oder, wenn erwünscht, Benzylgruppe, abspaltet und, wenn erwünscht, die entstehende OH-Gruppe durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, und, wenn erwünscht, eine erhaltene Verbindung der Formel I, in der A ein Halogenatom ist, mit einer Verbindung $NX_1X_2X_3$ umsetzt, oder eine erhaltene Verbindung der Formel I, in der A eine

$$\overset{\oplus}{N}H_3\text{-Gruppe}$$

ist, in eine Verbindung der Formel I, in der A eine

$$\overset{\oplus}{N}X_1X_2X_3\text{-Gruppe}$$

ist, überführt.

Ein Alkylrest R kann verzweigt oder vorzugsweise unverzweigt sein. Als Alkylrest besitzt R vorzugsweise 1 bis 7 C-Atome, in einem Acylrest —COR vorzugsweise 1 bis 3 oder 9 bis 19 C-Atome.

Eine Acylgruppe —COR leitet sich insbesondere von einer natürlichen Fettsäure, wie z. B. Behen-, Laurin-, Stearin-, Palmitin-, Myristin-, Caprin- oder Arachinsäure ab.

In einer Aralkylgruppe R ist der Arylrest vorzugsweise ein gegebenenfalls durch Alkyl mit 1 bis 7, insbesondere 1 oder 2 C-Atomen substituierter Phenylrest und die Alkylgruppe besitzt vorzugsweise 1 bis 4 C-Atome. Sie ist z. B. Phenäthyl und insbesondere Benzyl.

Ein Halogenatom A ist ein Fluor-, Chlor- oder Jodatom, insbesondere aber ein Bromatom.

Eine geradkettige oder verzweigte Alkylgruppe $X_1$, $X_2$ oder $X_3$ mit 1 bis 6 C-Atomen ist z. B. Äthyl, Propyl, Isopropyl, Butyl, Isobutyl, Hexyl usw., und insbesondere die Methylgruppe.

Die untereinander verschiedenen Reste $R_1$, $R_2$ und $R_3$ können sich auch nur in ihrer sterischen Konfiguration oder nur durch den Gehalt unterschiedlicher Isotopen, wie z.B.Deuterium, Tritium, $C_{14}$,unterscheiden. Vorzugsweise werden sn-Glycerinderivate hergestellt.

Die Abspaltung einer Tritylgruppe erfolgt unter schwach sauren Bedingungen, vorzugsweise bei einem pH-Wert von 4 bis 6, wobei der günstigste Wert unter Berücksichtigung der übrigen Substituenten im Molekül leicht eruiert werden kann. Die Allyl- und Benzylschutzgruppen sind hierbei vollkommen stabil. Die Reaktion kann in einem wäßrigen oder wäßrig-organischen, aber auch in einem rein organischen Medium, wie z. B. in absolutem Äthanol in Gegenwart von HCl, durchgeführt werden. Das organische Lösungsmittel kann dabei ein mit Wasser mischbares oder aber auch ein mit Wasser nur teilweise oder wenig mischbares inertes Lösungsmittel sein. Die Reaktion erfolgt, insbesondere beim Arbeiten in einem Zweiphasensystem, vorteilhafterweise unter starkem Rühren. Die Temperatur beträgt im allgemeinen 20 bis 80°C. Vorzugsweise erfolgt die Reaktion in 90%-igem Methanol, welches 10% in wss. HCl enthält, bei Rückflußtemperatur. Dabei kann es zur Verbesserung der Löslichkeit zweckmäßig sein, einen höheren Alkohol, insbesondere Propanol-(2), in kleiner Menge zuzusetzen.

Die Umlagerung der Allylgruppe in die Propenylgruppe geschieht vorzugsweise nach zwei verschiedenen Methoden, nämlich 1) unter alkalischen Bedingungen, wie z. B. mit Kalium-tert.-butylat in Dimethylformamid und anschließende Spaltung mit Brom in gepufferter Lösung bei einem pH-Wert um 5 bis 6, oder 2) durch Umlagerung in Gegenwart eines Palladium-auf-Kohle-Katalysators unter Bildung der unter diesen Bedingungen instabilen, spontan abspaltenden Propenylgruppe, wobei zweckmäßigerweise in 80%igem Methanol, welches in der wäßrigen Phase 20% Ameisensäure enthält, bei Rückflußtemperatur gearbeitet wird. Die Abspaltung erfolgt dabei unter Bedingungen, bei denen keine Wanderung von Acylgruppen oder Phosphorsäureresten eintritt.

Im allgemeinen ist die Variante 1), d. h. die Abspaltung mit Brom, bevorzugt. Zur Abspaltung der Propenylgruppe in 1-Stellung kann auch Jod verwendet werden (Eibl und Lands, Biochemistry *9* (1970) 423). Während aber eine Abspaltung der Propenylgruppe in 2-Stellung mit Jod überhaupt nicht möglich ist, läßt sich eine solche Abspaltung überraschenderweise mit Brom vollständig und in wenigen Minuten durchführen.

Die Abspaltung einer Benzylgruppe erfolgt durch katalytische Hydrogenolyse. Die Reaktionsbedingungen entsprechen dabei den üblichen Bedingungen. Insbesondere führt man die Hydrogenolyse in einem inerten Lösungsmittel, wie z. B. Äthanol, in Gegenwart eines Palladium- oder Platin/Palladium-Katalysators durch, vorzugsweise bei Raumtemperatur und unter Normaldruck (vgl. H. Eibl et al., Liebig's Ann. Chemie *738* (1970) 161).

Die Tetrahydropyranylgruppe kann unter schwach sauren Bedingungen in Gegenwart von Toluolsulfonsäure-Pyridiunium-Salz abgespalten werden. Diese Abspaltung wird beispielsweise bei einem

pH-Wert von 5 bis 6 durch Erhitzen in einer stark verdünnten wäßrigen Lösung von Toluolsulfonsäure-Pyridinium-Salz in Gegenwart von Borsäure (als Stabilisator) durchgeführt. Die Lösung enthält zweckmäßigerweise einen Zusatz eines organischen Lösungsvermittlers, wie z. B. Tetrahydrofuran oder Propanol-(2).

Die Acylierung einer freien OH-Gruppe kann durch Umsetzung mit einem entsprechenden Säureanhydrid der Formel $(RCO)_2O$ durchgeführt werden. Sie erfolgt, insbesondere zur Erzielung hoher Ausbeuten, vorteilhafterweise in Gegenwart von Perchlorsäure, wobei vorzugsweise mit einem ca. 10%-igen Überschuß an Anhydrid gearbeitet wird. Die Reaktionsbedingungen können dabei entsprechend der bekannten Acylierung von Acyl- oder Diacylglycerin gewählt werden (vgl. F.H. Mattson et al., J. Lipid Res. *5* (1964) 374). Es ist aber auch möglich, nach an sich bekannten anderen Acylierungsmethoden, z. B. wie von Gupta et al. (Proc. Nat. Acad. Sci. USA *74* (1977) 4315) beschieben, zu arbeiten.

Die Säureanhydride werden aus den entsprechenden Fettsäuren und Acetanhydrid hergestellt (D. Holde und K. Rietz, Ber. *57* (1924) 99).

Die Einführung einer Gruppe

$$-P(O)-O(CH_2)_yA,$$
$$\quad |$$
$$O^{\ominus}(Z^{\oplus})_n$$

worin A Amino, Halogen oder auch Wasserstoff bedeutet, und y, $Z^{\oplus}$ und n die oben angegebene Bedeutung besitzen, kann durch Umsetzung mit (Halogen)-Alkyl-phosphorsäuredichlorid (vgl. Hirt und Berchthold, Pharm. Acta Helv. *33* (1958) 349) oder vorzugsweise durch Umsetzung mit $POCl_3$ in Gegenwart von Triäthylamin und nachfolgende Umsetzung des erhaltenen Glycerin-phosphorsäure-dichlorids mit dem entsprechenden (Amino- oder Halogen)-alkanol (H. Eibl, Proc. Nat. Acad. Sci. USA *75* (1978) 4074; H. Eibl und A. Nicksch, Chem. Phys. Lipids *22* (1978) 1; W. Diembeck und H. Eibl, Chem. Phys. Lipids 24 (1979) 237) erfolgen.

Die Verätherung einer freien OH-Gruppe kann durch Umsetzung mit einem entsprechenden Halogenid, vorzugsweise Chlorid oder Bromid, unter den für eine solche Umsetzung üblichen bekannten Bedingungen erfolgen. Als Lösungsmittel können dabei die für derartige Umsetzungen üblichen Lösungsmittel, wie insbesondere z. B. Xylol oder Dioxan, eingesetzt werden. Die Reaktionstemperaturen liegen normalerweise über Raumtemperatur, vorzugsweise zwischen 50 und 100°C. Als Base werden Alkylate verwendet, insbesondere das Kalium-tert.-butylat. Zur Einführung langkettiger Alkylreste ist es zweckmäßig, anstelle der Halogenide die entsprechenden Mesylate zu verwenden.

Die Aminierung der Verbindungen der Formel I, worin A ein Halogenatom, vorzugsweise Brom, bedeutet, durch Umsetzung mit einem Amin $NX_1X_2X_3$ erfolgt in an sich bekannter Weise (vgl. H. Eibl und A. Nicksch*), Chem. Phys. Lipids *24* (1979) 237), ebenso die Alkylierung von Verbindungen der Formel I, worin A eine $NH_3$-Gruppe bedeutet, zu Verbindungen, in denen A eine

$$\overset{\oplus}{N}X_1X_2X_3\text{-Gruppe}$$

ist.

Als Ausgangsprodukt für die Herstellung der Verbindungen der Formel II dient vorzugsweise das 1,2- oder 2,3-Isoproyliden-glycerin (E. Baer, Biochem. Prep. *2* (1952) 31; J. Le Cocq und C. E. Ballou, Biochemistry *3* (1964) 976; C. M. Lok et al., Chem. Phys. Lipids *16* (1976) 115; H. Eibl, Chemistry and Physics of Lipids *28* (1981) 1). In die Isopropylidenverbindung wird zunächst durch Umsetzung mit Benzyl- oder Allylchlorid der Benzyl- oder Allylrest in die 1- oder 3-Stellung eingeführt, worauf die Isopropylidengruppe abgespalten wird (M. Kates et al., Biochemistry *2* (1963) 394). In die so erhaltene Verbindung wird dann in einer nachfolgenden Stufe die Tritylgruppe in 1- oder 3-Stellung durch Umsetzung mit Tritylchlorid in Gegenwart von Triäthylamin eingeführt. Nach Abdestillieren von Lösungsmittel und Triäthylamin wird das erhaltene Rohprodukt dann in tert.-Butanol gelöst und in Gegenwart von Kalium-tert.-butylat mit Allylchlorid oder Benzylchlorid zu den Verbindungen der Formel II umgesetzt.

Durch das erfindungsgemäße Verfahren werden die bekannten Nachteile der Synthese von Glycerinderivaten, wie z. B. Tri- oder Diacylglyerinen oder Glycerinphosphatiden, insbesondere die Wanderung der Acylreste und/oder des Phosphorsäurerestes, vermieden. Es hat sich gezeigt, daß die Verbindungen der Formel II sehr gute Ausgangsmaterialien zur Herstellung von Glycerinderivaten sind. Erfindungsgemäß können die einzelnen Schutzgruppen selektiv abgelöst werden, wobei jeder einzelne Verfahrensschritt unter Bedingungen durchgeführt werden kann, durch die die übrigen Substituenten nicht beeinflußt werden.

Zusammenfassend kann also festgestellt werden, daß das erfindungsgemäße Verfahren eine sehr selektive, einfache und wirtschaftliche Methode zur Herstellung von Glycerinderivaten darstellt, die vor allem für die stellungsspezifische Darstellung (Reinheit >98%) von Glycerinderivaten mit verschiedenen Resten von Bedeutung ist.

Die nachfolgenden Beispiele erläutern die Erfindung weiter, ohne sie darauf zu beschränken.

---

*)Chem. Phys. Lipids *22* (1978) 1; W. Diembeck und H. Eibl,

Beispiel 1

3-Benzyl-sn-glycerin

1,2-Isopropyliden-sn-glycerin (0,5 Mol), dargestellt nach Eibl (Chemistry and Physics of Lipids *28* (1981) 1—5) wird in 500 ml tert.-Butanol gelöst und mit Kalium-tert.-butylat (0,7 Mol) versetzt. Unter Rückfluß wird Benzylchlorid (0,6 Mol) eingetropft. Die Reaktion ist direkt nach dem Eintropfen beendet. Man filtriert und versetzt das Filtrat mit 500 ml Diisopropyläther und schüttelt gegen Wasser aus. Die obere Phase enthält das Produkt, wir einrotiert und mit 70%-iger Essigsäure (500 ml) versetzt. Nach 30 Minuten wird das Lösungsmittel entfernt und der Rückstand destelliert. $Kp_{1mm}$=145—7°C; $[\alpha]_D^{20}$=+6,6° (Literaturwert nur+5,5°).

Beispiel 2

3-Allyl-sn-glycerin

Entsprechend dem Verfahren von Beispiel 1 wird 3-Allyl-sn-glycerin dargestellt, mit Allylbromid als Alkylierungsmittel. Die physikalischen Daten für das 3-Allyl-sn-glycerin sind $Kp_{1mm}$=88°C; $[\alpha]_D^{20}$=+6,4°.

Beispiel 3

1-Trityl-2-allyl-3-benzyl-sn-glycerin

3-Benzyl-sn-glycerin (0,5 Mol) werden in 1 l tert.-Butanol gelöst und unter Rückfluß mit Tritylchlorid (0,6 Mol) in Gegenwart von Triäthylamin (0,7 Mol) dekocht. Nach 60 Minuten versetzt man mit jeweils 500 ml Diisopropyläther und Wasser. Die obere Phase wird einrotiert, der Rückstand (1-Trityl-3-benzyl-sn-glycerin) in tert.-Butanol gelöst und mit Kalium-tert.-butylat versetzt. Unter Rückfluß wird Allylchlorid eingetropft.

Beispiel 4

2-Allyl-3-benzyl-sn-glycerin

Das nach Beispiel 3 dargestellte 1-Trityl-2-allyl-3-benzyl-sn-glycerin wird in 1 l Methanol gelöst und unter Rückfluß mit 100 ml 1 n HCl versetzt. Die Tritylabspaltung ist nach 60 Minuten beendet. Man versetzt mit jeweils 1 l Diisopropyläther und Wasser. Die Diisopropylätherphase enthält das Produkt und wird mit 1N Natronlauge gewaschen und einrotiert. Der Rückstand wird chromatographisch gereinigt. Man erhält 2-Allyl-3-benzyl-sn-glycerin, Ausbeute 55%, $[\alpha]_D^{20}$=+6,0°.

Beispiel 5

1-Stearoyl-2-myristoyl-sn-glycerin

2-Allyl-3-benzyl-sn-glycerin wird mit Kalium-tert.-butylat in Dimethylformamid in 2-Propenyl-3-benzyl-sn-glycerin überführt. Das erhaltene Produkt wird in Gegenwart von 4-Dimethylamino-pyridin und Dicyclohexylcarbodiimid mit Stearinsäure verestert. Die Hydroxylgruppe in 2-Stellung wird durch Bromierung bei pH 5 (Phosphatpuffer) freigesetzt und in Gegenwart von 4-Dimethylaminopyridin und Dicyclohexylcarbodiimid mit Myristinsäure verestert. Das erhaltene 1-Stearoyl-2-myristoyl-3-benzyl-sn-glycerin wird einer katalytischen Hydrogenolyse in Gegenwart von Palladium unterworfen. Man erhält das 1-Stearoyl-2-myristoyl-sn-glycerin in einer Ausbeute von 78%, bezogen auf 2-Allyl-3-benzyl-sn-glycerin.

Auf analogue Weise wurden unter Verwendung der entsprechenden Fettsäuren hergestellt:

1-Myristoyl-2-stearoyl-sn-glycerin,

1-Behenoyl-2-myristoyl-sn-glycerin,

1-Behenzoyl-2-lauroyl-sn-glycerin und

die entsprechenden Derivate, in denen die Fettsäurereste in der 1- und 2-Stellung vertauscht sind.

Beispiel 6

1-Octadecyl-2-myristoyl-sn-glycerin

2-Allyl-3-benzyl-sn-glycerin wird mit Octadecylmesylat in Gegenwart von Kalium-tert.-butylat in tert.-Butanol alkyliert. Nach der Umlagerung der Allylgruppe in die Propenylgruppe mit Kalium-tert.-butylat in Dimethylformamid wird die Hydroxylgruppe in 2-Stellung durch Bromierung freigesetzt und wie in Beispiel 5 beschrieben mit Myristinsäure verestert. Nach katalytischer Hydrogenolyse in Gegenwart von Palladium wird das 1-Octadecyl-2-myristoyl-sn-glycerin in einer Ausbeute von 71%, bezogen auf 2-Allyl-3-benzyl-sn-glycerin, erhalten.

Beispiel 7

1-Stearoyl-3-benzyl-sn-glycerin

Ausgansverbindung: 2-Allyl-3-benzyl-sn-glycerin. Die Umlagerung von Allyl in Propenyl und die Veresterung mit Stearinsäure erfolgen wie in Beispiel 5 beschrieben. Nach Freisetzung der Hydroxylgruppe in 2-Stellung durch Bromierung bei pH 5 erhält man 1-Stearoyl-3-benzyl-sn-glycerin in einer Ausbeute von 67%, bezogen auf die Ausgangsverbindung.

Beispiel 8

1-Stearoyl-3-myristoyl-sn-glycerin

Ausgehend von 2-Benzyl-3-allyl-sn-glycerin erhält man nach der Reaktionsfolge von Beispiel 5 das 1-Stearoyl-3-myristoyl-sn-glycerin in einer Ausbeute von 74%, bezogen auf das Ausgangsprodukt.

Beispiel 9

1-Octadecyl-3-myristoyl-sn-glycerin

Ausgehend von 2-Benzyl-3-allyl-sn-glycerin erhält man nach der Reaktionsfolge von Beispiel 6 das 1-Octadecyl-3-myristoyl-sn-glycerin in einer Ausbeute von 79%, bezogen auf das Ausgangsprodukt.

Beispiel 10

1-Stearoyl-2-benzyl-sn-glycerin

Ausgehend von 2-Benzyl-3-allyl-sn-glycerin erhält man nach der Reaktionsfolge von Beispiel 7 das 1-Stearoyl-2-benzyl-sn-glycerin in einer Ausbeute von 76%, bezogen auf das Ausgangsprodukt.

Beispiel 11

1-Stearoyl-2-myristoyl-sn-glycero-3-phosphoäthanolamin

Eine Lösung von 1-Stearoyl-2-myristoyl-sn-glycerin in Tetrahydrofuran wird tropfenweise zu einer Lösung von Phosphoroxychlorid und Triäthanolamin in Tetrahydrofuran zugegeben. Eine Lösung des so erhaltenen 1-Stearoyl-2-myristoyl-sn-glycerin-3-phosphorsäuredichlorids in Tetrahydrofuran wird unter Rühren mit Äthanolamin in Gegenwart von Triäthylamin gesetzt. In dem dabei intermediär entstandenen 1,3,2-Oxazaphospholan wird der Ring an der P-N-Bindung unter schwach sauren Bedingungen (2-Propanol/wäßrige Ameisensäure) spezifisch gespalten. Man erhält 1-Stearoyl-2-myristoyl-sn-glycero-3-phosphoäthanolamin, weiße Kristalle, Ausbeute 90 bis 95%, bezogen auf 1-Stearoyl-2-myristoyl-sn-glycerin.

Beispiel 12

1-Stearoyl-2-myristoyl-sn-glycero-3-phosphocholin

Eine Lösung des nach Beispiel 11 erhaltene 1-Stearoyl-2-myristoyl-sn-glycero-3-phosphoäthanolamine in Chloroform/2-Propanol/Dimethylformamid und wäßrigem Natriumcarbonat wird mit einem Überschuß an Methyljodid umgesetzt. Unter diesen schwach alkalischen Methylierungsbedingungen wurde keine Zersetzung des Phosphatidylcholins beobachtet.

**Patentansprüche für die Vertragsstaaten: BE, CH, DE, FR, GB, IT, LI, LU, NL, SE**

1. Glycerinderivate der Formel

$$\begin{array}{l} CH_2{-}O{-}R_1' \\ | \\ CH{-}O{-}R_2' \\ | \\ CH_2{-}O{-}R_3', \end{array} \qquad (II)$$

worin jeder der Reste $R_1'$, $R_2'$ und $R_3'$ verschieden ist und eine Benzylgruppe oder Allylgruppe bedeutet, $R_1'$ und $R_3'$ auch eine Tritylgruppe.

2. 1-Trityl-2-allyl-3-benzyl-sn-glycerin.

3. 1-Trityl-2-benzyl-3-allyl-sn-glycerin.

4. Verfahren zur Herstellung von Glycerinderivaten der Formel II nach Anspruch 1, dadurch gekennzeichnet, daß man in ein 1- (oder 3-) Benzyl- (oder Allyl-)-glycerin in 3- (oder 1-)-Stellung die Tritylgruppe einführt und danach in die 2-Stellung die Allyl- (oder Benzyl-)-Gruppe.

5. Verfahren zur Weiterverarbeitung der Verbindungen der Formel II nach Anspruch 1 zur Herstellung von Glycerinderivaten der Formel I

$$\begin{array}{l} CH_2{-}O{-}R_1 \\ | \\ CH{-}O{-}R_2 \\ | \\ CH_2{-}O{-}R_3 \end{array} \qquad (I)$$

worin jeder der Reste $R_1$, $R_2$ und $R_3$ verschieden ist und einen gesättigten Alkylrest R, einen gesättigten Acylrest —COR oder Wasserstoff darstellt, wobei R eine geradkettige oder verzweigte Alkylgruppe mit 1 bis 25 C-Atomen bedeutet, oder einen Arylalkylrest mit 1 bis 25 C-Atomen in der Alkylkette, und worin zwei der Reste auch ungesättigt sein können, und $R_3$ auch die Gruppe

$$\begin{array}{l} P(O){-}O{-}(CH_2)_y{-}A \\ | \\ O^{\ominus}(Z^{\oplus})_n \end{array}$$

bedeuten kann, worin

A ein Halogenatom, die Gruppe

$$\overset{\oplus}{N}X_1X_2X_3$$

oder auch Wasserstoff ist, $X_1$, $X_2$ und $X_3$ gleich oder verschieden sind und Wasserstoff oder eine geradkettige oder verzweigte Alkylgruppe mit 1 bis 6 C-Atomen bedeuten,

y eine ganze Zahl von 2 bis 12 oder, wenn A ein Wasserstoffatom ist, auch O sein kann,

$Z^{\oplus}$ ein Äquivalent eines ein- bis dreiwertigen Kations ist und

n 1 oder, wenn A die Gruppe

$$\overset{\oplus}{N}X_1X_2X_3$$

bedeutet, O ist, und worin, wenn A die Gruppe

$$\overset{\oplus}{N}X_1X_2X_3$$

bedeutet, $-(CH_2)_y-$ auch durch eine Carboxylgruppe substituiert sein kann,

dadurch gekennzeichnet, daß man von Verbindungen der Formel II nach Anspruch 1 ausgeht, und, wenn die Reste $R_1$, $R_2$ und $R_3$ gesättigt sind, oder nur einer der Reste ungesättigt ist,

a) die Tritylgruppe unter schwach sauren Bedingungen abspaltet und die dadurch entstehende OH-Gruppe dann durch Verätherung (für einen von Benzylverschiedenen Arylalkylrest) oder Acylierung in eine gesättigte Gruppe $OR_1$ oder $OR_3$ überführt oder durch Phosphorilierung in eine Gruppe $OR_3$, danach

b) die Allylgruppe in die Propylengruppe umlagert und diese abspaltet, und die dadurch entstehende OH-Gruppe, wenn erwünscht, dann durch Verätherung (für einen von Benzyl verschiedenen Arylalkylrest) oder Acylierung in eine gesättigte Gruppe $OR_1$, $OR_2$ oder $OR_3$ oder durch Phosphorilierung in eine Gruppe $OR_3$ überführt, wobei die Umlagerung der Allylgruppe in die Propenylgruppe auch bereits in der Stufe a) vor der Verätherung, Acylierung oder Phosphorilierung erfolgen kann, und anschließend, wenn erwünscht,

c) die Benzylgruppe durch katalytische Hydrogenolyse entfernt und die dadurch entstehende OH-Gruppe, wenn erwünscht, durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, oder, wenn die Reste gesättigt oder ungesättigt sind,

$a_1$) die Tritylgruppe abspaltet und die entstehende OH-Gruppe mit einer Tetrahydropyranylgruppe veräthert, danach

$b_1$) die Allylgruppe abspaltet und die entstehende OH-Gruppe durch Verätherung (für einen von Benzyl verschiedenen Arylalkylrest) oder Acylierung in eine gesättigte Gruppe $OR_1$, $OR_2$ oder $OR_3$ oder durch Phosphorilierung in eine Gruppe $OR_3$ überführt,

$c_1$) danach die Tetrahydropyranylgruppe oder, wenn erwünscht, die Benzylgruppe abspaltet und die entstehende OH-Gruppe durch Verätherung (für einen von Benzyl verschiedenen Arylalkylrest) oder Acylierung in eine gesättigte (nach Abspaltung der Tetrahydropyranylgruppe) oder gesättigte oder ungesättigte (nach Abspaltung der Benzylgruppe) Gruppe $OR_1$, $OR_2$ oder $OR_3$ oder durch Phosphorilierung in eine Gruppe $OR_3$ überführt und anschließend

$d_1$) die verbleibende Tetrahydropyranylgruppe, oder, wenn erwünscht, Benzylgruppe abspaltet und, wenn erwünscht, die entstehende OH-Gruppe durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, oder

$a_2$) die Tritylgruppe abspaltet und die entstehende OH-Gruppe mit einer langkettigen gesättigten Fettsäure mit 10 bis 25 C-Atomen acyliert, danach

$b_2$) die Allylgruppe abspaltet und die entstehende OH-Gruppe mit einer Tetrahydropyranylgruppe veräthert und

$c_2$) wenn erwünscht, den langkettigen Acylrest abspaltet und die entstehende OH-Gruppe durch Verätherung (für einen von Benzyl verschiedenen Arylalkylrest) oder Acylierung in eine gesättigte Gruppe $OR_1$ oder $OR_3$ oder durch Phosphorilierung in eine Gruppe $OR_3$ überführt, und danach

$d_2$) die Tetrahydropyranylgruppe oder, wenn erwünscht, die Benzylgruppe abspaltet und die entstehende OH-Gruppe durch Verätherung (für einen von Benzyl verschiedenen Arylalkylrest) oder Acylierung in eine gesättigte (nach Abspaltung der Tetrahydropyranylgruppe) oder gesättigte oder ungesättigte (nach Abspaltung der Benzylgruppe) Gruppe $OR_1$, $OR_2$ oder $OR_3$ oder durch Phosphorilierung in eine Gruppe $OR_3$ überführt und anschließend

$e_2$) die verbleibende Tetrahydropyranylgruppe oder, wenn erwünscht, Benzylgruppe abspaltet und, wenn erwünscht, die entstehende OH-Gruppe durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_2$ überführt, und, wenn erwünscht, eine erhaltene Verbindung der Formel I, in der A ein Halogenatom ist, mit einer Verbindung

$$\overset{\oplus}{N}X_1X_2X_3$$

umsetzt, oder eine erhaltene Verbindung der Formel I, in der A eine

$\overset{\oplus}{N}H_3$-Gruppe

ist, in eine Verbindung der Formel I, in der A eine

$\overset{\oplus}{N}X_1X_2X_3$-Gruppe

ist, überführt.

6. Verfahren nach Anspruch 5, dadurch gekennzeichnet, daß der Alkylrest R im Rest —COR 1 bis 3 C-Atome besitzt.

7. Verfahren nach Anspruch 5, dadurch gekennzeichnet, daß der Alkylrest R im Rest —COR 9 bis 19 C-Atome besitzt.

8. Verfahren nach Anspruch 5 oder 7, dadurch gekennzeichnet, daß die Acylgruppe der Rest einer natürlichen Fettsäure ist.

9. Verfahren nach einem der Ansprüche 5 bis 8, dadurch gekennzeichnet, daß man von 1-Trityl-2-allyl-3-benzyl-sn-glycerin ausgeht.

10. Verfahren nach einem der Ansprüche 5 bis 8, dadurch gekennzeichnet, daß man von 1-Trityl-2-benzyl-3-allyl-sn-glycerin ausgeht.

**Patentansprüche für den Vertragsstaat: AT**

1. Verfahren zur Herstellung von Glycerinderivaten der Formel II

$$\begin{array}{l} CH_2—O—R_1' \\ | \\ CH—O—R_2' \\ | \\ CH_2—O—R_3', \end{array} \qquad (II)$$

worin jeder der Reste $R_1'$, $R_2'$ und $R_3'$ verschieden ist und eine Benzylgruppe oder Allylgruppe bedeutet, $R_1'$ und $R_3'$ auch eine Tritylgruppe, indem man in ein 1- (oder 3-) Benzyl (oder Allyl-)-glycerin in 3- (oder 1-)-Stellung die Tritylgruppe einführt und danach in die 2-Stellung die Allyl- (oder Benzyl-)-Gruppe.

2. Verfahren zur Weiterverarbeitung der gemäß dem Verfahren von Anspruch 1 hergstellten Verbindungen der Formel II zur Herstellung von Glycerinderivaten der Formel I

$$\begin{array}{l} CH_2—O—R_1 \\ | \\ CH—O—R_2 \\ | \\ CH_2—O—R_3 \end{array} \qquad (I)$$

worin jeder der Reste $R_1$, $R_2$ und $R_3$ verschieden ist und einen gesättigten Alkylrest R, einen gesättigten Acylrest —COR oder Wasserstoff darstellt, wobei R eine geradkettige oder verzweigte Alkylgruppe mit 1 bis 25 C-Atomen bedeutet, oder einen Arylalkylrest mit 1 bis 25 C-Atomen in der Alkylkette, und worin zwei der Reste auch ungesättigt sein können, und $R_3$ auch die Gruppe

$$\begin{array}{l} P(O)—O—(CH_2)_y—A \\ | \\ O^{\ominus}(Z^{\oplus})_n \end{array}$$

bedeuten kann, worin

A ein Halogenatom, die Gruppe

$\overset{\oplus}{N}X_1X_2X_3$

oder auch Wasserstoff ist, $X_1$, $X_2$ und $X_3$ gleich oder verschieden sind und Wasserstoff oder eine geradkettige oder verzweigte Alkylgruppe mit 1 bis 6 C-Atomen bedeuten,

y eine ganze Zahl von 2 bis 12 oder, wenn A ein Wasserstoffatom ist, auch O sein kann,

$Z^{\oplus}$ ein Äquivalent eines ein- bis dreiwertigen Kations ist und

n 1 oder, wenn A die Gruppe

$\overset{\oplus}{N}X_1X_2X_3$

bedeutet, O ist, und worin, wenn A die Gruppe

$$\overset{\oplus}{N}X_1X_2X_3$$

bedeutet, $-(CH_2)_y-$ auch durch eine Carboxylgruppe substituiert sein kann,

dadurch gekennzeichnet, daß man von Verbindungen der Formel II nach Anspruch 1 ausgeht, und, wenn die Reste $R_1$, $R_2$ und $R_3$ gesättigt sind, oder nur einer der Reste ungesättigt ist,

a) die Tritylgruppe unter schwach sauren Bedingungen abspaltet und die dadurch entstehende OH-Gruppe dann durch Verätherung (für einen von Benzyl verschiedenen Arylalkylrest) oder Acylierung in eine gesättigte Gruppe $OR_1$ oder $OR_3$ überführt oder durch Phosphorilierung in eine Gruppe $OR_3$, danach

b) die Allylgruppe in die Propylengruppe umlagert und diese abspaltet, und die dadurch entstehende OH-Gruppe, wenn erwünscht, dann durch Verätherung (für einen von Benzyl verschiedenen Arylalkylrest) oder Acylierung in eine gesättigte Gruppe $OR_1$, $OR_2$ oder $OR_3$ oder durch Phosphorilierung in eine Gruppe $OR_3$ überführt, wobei die Umlagerung der Allylgruppe in die Propenylgruppe auch bereits in der Stufe a) vor der Verätherung, Acylierung oder Phosphorilierung erfolgen kann, und anschließend, wenn erwünscht,

c) die Benzylgruppe durch katalytische Hydrogenolyse entfernt und die dadurch entstehende OH-Gruppe, wenn erwünscht, durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, oder, wenn die Reste gesättigt oder ungesättigt sind,

$a_1$) die Tritylgruppe abspaltet und die entstehende OH-Gruppe mit einer Tetrahydropyranylgruppe veräthert, danach

$b_1$) die Allylgruppe abspaltet und die entstehende OH-Gruppe durch Verätherung (für einen von Benzyl verschiedenen Arylalkylrest) oder Acylierung in eine gesättigte Gruppe $OR_1$, $OR_2$ oder $OR_3$ oder durch Phosphorilierung in eine Gruppe $OR_3$ überführt,

$c_1$) danach die Tetrahydropyranylgruppe oder, wenn erwünscht, die Benzylgruppe abspaltet und die entstehende OH-Gruppe durch Verätherung (für einen von Benzyl verschiedenen Arylalkylrest) oder Acylierung in eine gesättigte (nach Abspaltung der Tetrahydropyranylgruppe) oder gesättigte oder ungesättigte (nach Abspaltung der Benzylgruppe) Gruppe $OR_1$, $OR_2$ oder $OR_3$ oder durch Phosphorilierung in eine Gruppe $OR_3$ überführt und anschließend

$d_1$) die verbleibende Tetrahydropyranylgruppe, oder, wenn erwünscht, Benzylgruppe abspaltet und, wenn erwünscht, die entstehende OH-Gruppe durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, oder

$a_2$) die Tritylgruppe abspaltet und die entstehende OH-Gruppe mit einer langkettigen gesättigten Fettsäure mit 10 bis 25 C-Atomen acyliert, danach

$b_2$) die Allylgruppe abspaltet und die entstehende OH-Gruppe mit einer Tetrahydropyranylgruppe veräthert und

$c_2$) wenn erwünscht, den langkettigen Acylrest abspaltet und die entstehende OH-Gruppe durch Verätherung (für einen von Benzyl verschiedenen Arylalkylrest) oder Acylierung in eine gesättigte Gruppe $OR_1$ oder $OR_3$ oder durch Phosphorilierung in eine Gruppe $OR_3$ überführt, und danach

$d_2$) die Tetrahydropyranylgruppe oder, wenn erwünscht, die Benzylgruppe abspaltet und die entstehende OH-Gruppe durch Verätherung (für einen von Benzyl verschiedenen Arylalkylrest) oder Acylierung in eine gesättigte (nach Abspaltung der Tetrahydropyranylgruppe) oder gesättigte oder ungesättigte (nach Abspaltung der Benzylgruppe) Gruppe $OR_1$, $OR_2$ oder $OR_3$ oder durch Phosphorilierung in eine Gruppe $OR_3$ überführt und anschließend

$e_2$) die verbleibende Tetrahydropyranylgruppe oder, wenn erwünscht, Benzylgruppe abspaltet und, wenn erwünscht, die entstehende OH-Gruppe durch Verätherung (für einen von Benzyl verschiedenen Arylalkylrest) oder Acylierung in eine gesättigte oder ungesättigte Gruppe $OR_1$, $OR_2$ oder $OR_3$ oder durch Phosphorilierung in eine Gruppe $OR_3$ überführt, und, wenn erwünscht, eine erhaltene Verbindung der Formel I, in der A ein Halogenatom ist, mit einer Verbindung

$$\overset{\oplus}{N}X_1X_2X_3$$

umsetzt, oder eine erhaltene Verbindung der Formel I, in der A eine

$$\overset{\oplus}{N}H_3\text{-Gruppe}$$

ist, in eine Verbindung der Formel I, in der A eine

$$\overset{\oplus}{N}X_1X_2X_3\text{-Gruppe}$$

ist, überführt.

3. Verfahren nach Anspruch 2, dadurch gekennzeichnet, daß der Alkylrest R im Rest —COR 1 bis 3 C-Atome besitzt.

4. Verfahren nach Anspruch 2, dadurch gekennzeichnet, daß der Alkylrest R im Rest —COR 9 bis 19 C-Atome besitzt.

5. Verfahren nach Anspruch 2 oder 4, dadurch gekennzeichnet, daß die Acylgruppe der Rest einer natürlichen Fettsäure ist.

6. Verfahren nach einem der Ansprüche 2 bis 5, dadurch gekennzeichnet, daß man von 1-Trityl-2-allyl-3-benzyl-sn-glycerin ausgeht.

7. Verfahren nach einem der Ansprüche 2 bis 5, dadurch gekennzeichnet, daß man von 1-Trityl-2-benzyl-3-allyl-sn-glycerin ausgeht.

**Revendications pour les Etats Contractants: BE, CH, DE, FR, GB, IT, LI, LU, NL, SE**

1. Dérivés du glycérol répondant à la formule

$$\begin{array}{l} CH_2{-}O{-}R_1' \\ | \\ CH{-}O{-}R_2' \\ | \\ CH_2{-}O{-}R_3', \end{array} \qquad (II)$$

dans laquelle chacun des radicaux $R_1'$, $R_2'$ et $R_3'$ est différent et désigne un groupe benzyle ou un groupe allyle, $R_1'$ et $R_3'$ pouvant également désigner un groupe trityle.

2. 1-trityl-2-allyl-3-benzyl-sn-glycérol.

3. 1-trityl-2-benzyl-3-allyl-sn-glycérol.

4. Procédé de préparation de dérivés du glycérol répondant à la formule II suivant la revendication 1, caractérisé en ce qu'on introduit le groupe trityle dans un 1- (ou 3-) benzyl- (ou allyl-) -glycérol en position 3- (ou 1-) puis le groupe allyl- (ou benzyl-) en position 2.

5. Procédé de traitement ultérieur des composés répondant à la formule II suivant la revendication 1, pour la préparation de dérivés du glycérol répondant à la formule I

$$\begin{array}{l} CH_2{-}O{-}R_1 \\ | \\ CH{-}O{-}R_2 \\ | \\ CH_2{-}O{-}R_3 \end{array} \qquad (I)$$

dans laquelle chacun des radicaux $R_1$, $R_2$ et $R_3$ est différent et désigne un radical alkyle saturé R, und radical acyle saturé —COR ou l'hydrogène, R pouvant être un groupe alkyle linéaire ou ramifié ayant de 1 à 25 atomes de C, ou un radical arylalkyle ayant de 1 à 25 atomes de carbone dans la chaîne alkyle, et dans laquelle deux des radicaux peuvent également être insaturés, et $R_3$ peut aussi désigner le groupe

$$\begin{array}{l} P(O){-}O{-}(CH_2)_y{-}A \\ | \\ O^{\ominus}(Z^{\oplus})_n \end{array}$$

où

A est un atome d'halogène, le groupe

$$\overset{\oplus}{N}X_1X_2X_3$$

ou encore l'hydrogène,

$X_1$, $X_2$, $X_3$ sont identiques ou différents et désignant l'hdyrogène ou un groupe alkyle linéaire ou ramifié ayant de 1 à 6 atomes de C,

y est un nombre entier de 2 à 12 ou peut aussi être O lorsque A est un atome d'hydrogène,

$Z^{\oplus}$ est un équivalent d'un cation mono à trivalent et,

n est 1 ou, lorsque A est le groupe

$$\overset{\oplus}{N}X_1X_2X_3$$

O, et où, lorsque A désigne le groupe

$$\overset{\oplus}{N}X_1X_2X_3$$

$-(CH_2)_y-$ peut aussi être substitué par un groupe carboxyle,

caractérisé en ce qu'on part de composés répondant à la formule II suivant la revendication 1, et lorsque les radicaux $R_1$, $R_2$ et $R_3$ sont saturés, ou 1 seul des radicaux est insaturé,

a) on enlève par scission le groupe trityle dans des conditions faiblement acides, et on transforme ensuite le groupe OH formé, par éthérification (pour un radical arylalkyle différent du radical benzyle) ou par acylation, en un groupe saturé $OR_1$ ou $OR_3$ ou par phosphorylation en un groupe $OR_3$, puis

b) on transpose le groupe allyle en groupe propylène, et l'on enlève par scission ce groupe propylène, on transforme ensuite si on le désire le groupe OH ainsi formé par éthérification (pour un radical arylalkyle différent du radical benzyle) ou acylation, en un groupe saturé $OR_1$, $OR_2$ ou $OR_3$ ou par phosphorylation en un groupe $OR_3$, la transposition du groupe allyle en groupe propényle pouvant s'effectuer déjà au stade a) avant l'éthérification, l'acylation ou la phosphorylation, puis, si on le désire

c) on élimine le groupe benzyle par hydrogénolyse catalytique, et, si on le désire on transformé le groupe OH ainsi formé par éthérification (pour un radical arylalkyle différent du radical benzyle) ou acylation, en un groupe saturé ou insaturé $OR_1$, $OR_2$ ou $OR_3$, ou par phosphorylation en un groupe $OR_3$, ou, lorsque les radicaux sont saturés ou insaturés,

$a_1$) on enlève par scission le groupe trityle et on éthérifie le groupe OH ainsi formé avec un groupe tétrahydropyranyle, puis

$b_1$) on enlève par scission le groupe allyle, et on transforme le groupe OH formé, par éthérification (pour un radical arylalkyle différent du radical benzyle) ou acrylation, en un groupe saturé $OR_1$, $OR_2$ ou $OR_3$, ou par phosphorylation en un groupe $OR_3$,

$c_1$) puis on enlève par scission le groupe tétrahydropyranyle, ou, si on le désire, le groupe benzyle, et on transforme le groupe OH formé par éthérification (pour un radical arylalkyle différent du radical benzyle) ou acylation, en un groupe saturé (après enlèvement par scission du groupe tétrahydropyranyle) ou saturé ou insaturé (après enlèvement par scission du groupe benzyle) $OR_1$, $OR_2$ ou $OR_3$, ou par phosphorylation, en un groupe $OR_3$, puis

$d_1$) on enlève par scission le groupe tétrahydropyranyle restant ou, si on le désire, le groupe benzyle et, si on le désire, on transforme le groupe OH formé par éthérification (pour un radical arylalkyle différent du radical benzyle) ou acylation, en un groupe saturé ou insaturé $OR_1$, $OR_2$ ou $OR_3$, ou par phosphorylation en un groupe $OR_3$, ou

$a_2$) on enlève par scission le groupe trityle, et on acyle le groupe OH formé par un acide gras saturé à longue chaîne ayant de 10 à 25 atomes de C, puis

$b_2$) on enlève par scission le groupe allyle et on éthérifie le groupe OH formé avec un groupe tétrahydropyranyle, et

$c_2$) si on le désire, on enlève par scission le radical acyle à longue chaîne et on transforme le groupe OH formé par éthérification (pour un radical arylalkyle différent du radical benzyle) ou acylation en un groupe saturé $OR_1$ ou $OR_3$, ou par phosphorylation, en un groupe $OR_3$, puis

$d_2$) on enlève par scission le groupe tétrahydropyranyle ou, si on le désire, le groupe benzyle, et on transforme le groupe OH formé par éthérification (pour un radical arylalkyle différent du radical benzyle) ou acrylation en un groupe saturé (après enlèvement par scission du groupe tétrahydropyranyle) ou saturé ou insaturé (après enlèvement par scission du groupe benzyle) $OR_1$, $OR_2$ ou $OR_3$, ou par phosphorylation en un groupe $OR_3$, puis

$e_2$) on enlève par scission le groupe tétrahydropyranyle restant ou, si on le désire, le groupe benzyle, et, si on le désire, on transforme le groupe OH formé par éthérification (pour un radical arylalkyle différent du radical benzyle) ou acrylation en un groupe saturé ou insaturé $OR_1$, $OR_2$ ou $OR_3$, ou par phosphorylation en un groupe $OR_3$, et, si on le désire, on fait réagir un composé obtenu répondant à la formule I, dans laquelle A est un atome d'halogène, avec un composé

$$\overset{\oplus}{N}X_1X_2X_3$$

ou on transforme un composé obtenu répondant à la formule I, dans laquelle A désigne un groupe

$$\overset{\oplus}{N}H_3$$

en un composé répondant à la formule I, dans laquelle A est un groupe

$$\overset{\oplus}{N}X_1X_2X_3.$$

6. Procédé suivant la revendication 5, caractérisé en ce que le radical alkyle R dans le radical —COR possède 1 à 3 atomes de carbone.

7. Procédé suivant la revendication 5, caractérisé en ce que le radical alkyle R dans le radical —COR possède 9 à 19 atomes de carbone.

8. Procédé suivant les revendications 5 ou 7 caractérisé en ce que le groupe acyle est le radical d'un acide gras naturel.

9. Procédé suivant l'une des revendications 5 à 8, caractérisé en ce qu'on part du 1-trityl-2-allyl-3-benzyl-sn-glycérol.

10. Procédé suivant l'une des revendication 5 à 8, caractérisé en ce qu'on part du 1-trityl-2-benzyl-3-allyl-sn-glycérol.

**Revendications pour l'Etat Contractant: AT**

1. Procédé de préparation de dérivés du glycérol répondant à la formule II

$$\begin{array}{l} CH_2-O-R_1' \\ | \\ CH-O-R_2' \\ | \\ CH_2-O-R_3', \end{array} \qquad (II)$$

dans laquelle chacun des radicaux $R_1'$, $R_2'$ et $R_3'$ est différent et désigne un groupe benzyle ou un groupe allyle, $R_1'$ et $R_3'$ pouvant également désigner un groupe trityle, en introduisant dans un 1- (ou 3-) benzyl- (ou allyl-) -glycérol en position 3- (ou 1-) le groupe trityle, puis en position 2 le groupe allyl- (ou benzyl-).

2. Procédé de traitement ultérieur des composés répondant à la formule II préparés conformément au procédé de la revendication 1, pour la préparation de dérivés du glycérol répondant à la formule I

$$\begin{array}{l} CH_2-O-R_1 \\ | \\ CH-O-R_2 \\ | \\ CH_2-O-R_3 \end{array} \qquad (I)$$

dans laquelle chacun des radicaux $R_1$, $R_2$ et $R_3$ est différent et représente un radical alkyle saturé R, un radical acyle saturé —COR ou l'hydrogène, R désignent un groupe alkyle ayant de 1 à 25 atomes de C linéaire ou ramifié, ou un radical arylalkyle ayant de 1 à 25 atomes de carbone dans la chaîne alkyle, et dans laquelle deux des radicaux peuvent aussi être insaturés, et $R_3$ peut désigner également le groupe

$$\begin{array}{l} P(O)-O-(CH_2)_y-A \\ | \\ O^{\ominus}(Z^{\oplus})_n \end{array}$$

dans lequel

A est un atome d'halogène, le groupe

$$\overset{\oplus}{N}X_1X_2X_3$$

ou encore l'hydrogène,

$X_1$, $X_2$, et $X_3$ sont identiques ou différents et désignant l'hydrogène ou un groupe alkyle linéaire ou ramifié ayant de 1 à 6 atomes de C,

y est un nombre entier de 2 à 12 ou, lorsque A est un atome d'hydrogène, peut également être O,

$Z^{\oplus}$ est un équivalent d'un cation mono à trivalent, et

n est 1 ou, lorsque A désigne le groupe

$$\overset{\oplus}{N}X_1X_2X_3$$

O, et dans lequel, lorsque A désigne le groupe

$$\overset{\oplus}{N}X_1X_2X_3$$

$-(CH_2)_y-$ peut aussi être substitué par un groupe carboxyle,

caractérisé en ce qu'on part de composés répondant à la formule II suivant la revendication 1, et lorsque les radicaux $R_1$, $R_2$ et $R_3$ sont saturés, ou que seul un des radicaux est insaturé,

a) on enlève par scission le groupe trityle dans des conditions faiblement acides, et on transforme ensuite le groupe OH ainsi formé, par éthérification (pour un radical arylalkyle différent du radical benzyle) ou par acylation, en un groupe saturé $OR_1$ ou $OR_3$ ou par phosphorylation en un groupe $OR_3$, puis

b) on transpose le groupe allyle en le groupe propylène, et l'on enlève par scission ce groupe propylène, puis on transforme le groupe OH ainsi formé, par éthérification (pour un radical arylalkyle différent du radical benzyle) ou acylation, en un groupe saturé $OR_1$, $OR_2$ ou $OR_3$ ou par phosphorylation en un groupe $OR_3$, la transposition du groupe allyle en groupe propényle pouvant déjà effectuée au stade a) avant l'éthérification, l'acylation ou la phosphorylation, puis, si on le désire

c) on élimine le groupe benzyle par hydrogénolyse catalytique, et, on transforme si on le désire le groupe OH ainsi formé par éthérification (pour un radical arylalkyle différent du radical benzyle) ou acylation, en un groupe saturé ou insaturé $OR_1$, $OR_2$ ou $OR_3$, ou par phosphorylation en un groupe $OR_3$, ou, lorsque les radicaux sont saturée ou insaturés,

$a_1$) on enlève par scission le groupe trityle et on éthérifie le groupe OH formé avec un groupe tétrahydropyranyle, puis

$b_1$) on enlève par scission le groupe allyle, et on transforme le groupe OH formé par éthérification (pour un radical arylalkyle différent du radical benzyle) ou acrylation, en un groupe saturé $OR_1$, $OR_2$ ou $OR_3$, ou par phosphorylation en un groupe $OR_3$,

$c_1$) puis on enlève par scission le groupe tétrahydropyranyle, ou, si on le désire, le groupe benzyle, et on transforme le groupe OH formé par éthérification (pour un radical arylalkyle différent du radical benzyle) ou acylation, en un groupe saturé (après enlèvement par scission du groupe tétrahydropyranyle) ou saturé ou insaturé (après enlèvement par scission du groupe benzyle) $OR_1$, $OR_2$ ou $OR_3$, ou par phosphorylation, en un groupe $OR_3$, puis

$d_1$) on enlève par scission le groupe tétrahydropyranyle restant ou, si on le désire, le groupe benzyle et, si on le désire, on transforme le groupe OH formé par éthérification (pour un radical arylalkyle différent du radical benzyl) ou acylation, en un groupe saturé ou insaturé $OR_1$, $OR_2$ ou $OR_3$, ou par phosphorylation en un groupe $OR_3$, ou

$a_2$) on enlève par scission le groupe trityle, et on acyle le groupe OH formé avec un acide gras saturé à longue chaîne ayant de 10 à 25 atomes de C, puis

$b_2$) on enlève par scission le groupe allyle et on éthérifie le groupe OH formé avec un groupe tétrahydropyranyle, et

$c_2$) si on le désire, on enlève par scission le radical acyle à longue chaîne et on transforme le groupe OH formé par éthérification (pour un radical arylalkyle différent du radical benzyle) ou acylation en un groupe saturé $OR_1$ our $OR_3$, ou par phosphorylation, en un groupe $OR_3$, puis

$d_2$) on enlève par scission le groupe tétrahydropyranyle ou, si on le désire, le groupe benzyle, et on transforme le groupe OH formé par éthérification (pour un radical arylalkyle différent d'un radical benzyle) ou acylation en un groupe saturé (après enlèvement par scission du groupe têtrahydropyranyle) ou sáturé ou insaturé (après enlèvement par scission du groupe benzyle) $OR_1$, $OR_2$ ou $OR_3$, ou par phosphorylation en un groupe $OR_3$, puis

$e_2$) on enlève par scission le groupe tétrahydropyranyle restant ou, si on le désire, le groupe benzyle, et, si on le désire, on transforme le groupe OH formé par éthérification (pour un radical arylalkyle différent du radical benzyle) ou acrylation en un groupe saturé ou insaturé $OR_1$, $OR_2$ ou $OR_3$, ou par phosphorylation en un groupe $OR_3$, et, si on le désire, on fait réagir un composé obtenu répondant à la formule I, dans laquelle A est un atome d'halogène, avec un composé

$$\overset{\oplus}{N}X_1X_2X_3$$

ou on transforme un composé obtenu répondant à la formule I, dans laquelle A est un groupe

$$\overset{\oplus}{N}H_3$$

en un composé répondant à la formule I, dans laquelle A est un groupe

$$\overset{\oplus}{N}X_1X_2X_3.$$

3. Procédé suivant la revendication 2, caractérisé en ce que le radical alkyle R du radical —COR possède 1 à 3 atomes de carbone.

4. Procédé suivant la revendication 2, caractérisé en ce que le radical alkyle R dans le radical —COR possède 9 à 19 atomes de carbone.

5. Procédé suivant les revendications 2 ou 4, caractérisé en ce que le groupe acyle est le radical d'un acide gras naturel.

6. Procédé suivant l'une des revendications 2 à 5, caractérisé en ce qu'on part du 1-trityl-2-allyl-3-benzyl-sn-glycérol.

7. Procédé suivant l'une des revendications 2 à 5, caractérisé en ce qu'on part du 1-trityl-2-benzyl-3-allyl-sn-glycérol.

**Claims for the Contracting States: BE, CH, DE, FR, GB, IT, LI, LU, NL, SE**

1. Glycerol derivatives of the formula:—

$$\begin{array}{l} CH_2—O—R'_1 \\ | \\ CH—O—R'_2 \\ | \\ CH_2—O—R'_3 \end{array} \qquad (II)$$

wherein each of the radicals $R'_1$, $R'_2$ and $R'_3$ is different and signifies a benzyl group or allyl group, $R'_1$ and $R'_3$ also a trityl group.

2. 1-Trityl-2-allyl-3-benzyl-sn-glycerol.

3. 1-Trityl-2-benzyl-3-allyl-sn-glycerol.

4. Process for the preparation of glycerol derivatives of formula II according to claim 1, characterised in that into a 1- (or 3-) benzyl- (or allyl-) glycerol one introduces the trityl group into the 3- (or 1-) position and thereafter the allyl (or benzyl) group into the 2-position.

5. Process for the further working up of the compounds of formula II according to claim 1 for the preparation of glycerol derivatives of formula I:

$$\begin{array}{l} CH_2{-}O{-}R_1 \\ | \\ CH{-}O{-}R_2 \\ | \\ CH_2{-}O{-}R_3 \end{array} \qquad (I)$$

wherein each of the radicals $R_1$, $R_2$ and $R_3$ is different and represents a saturated alkyl radical R, a saturated acyl radical —COR or hydrogen, whereby R signifies a straight-chained or branched alkyl group with 1 to 25 C-atoms or an arylalkyl radical with 1 to 25 C-atoms in the alkyl chain and wherein two of the radicals can also be unsaturated and $R_3$ can also signify the group

$$\begin{array}{l} -P(O){-}O{-}(CH_2)_y{-}A \\ \;\;| \\ \;\;O^{\ominus}(Z^{\oplus})_n \end{array}$$

wherein A is a halogen atom, the group

$$-\overset{\oplus}{N}X_1X_2X_3$$

or also hydrogen, $X_1$, $X_2$ and $X_3$ are the same or different and signify hydrogen or a straight-chained or branched alkyl group with 1 to 6 C-atoms, y is a whole number from 2 to 12 or, when A is a hydrogen atom, can also be O, $Z^{\oplus}$ is an equivalent of a mono- to trivalent cation and n is 1 or, when A signifies the group

$$\overset{\oplus}{N}X_1X_2X_3,$$

is 0 and wherein, when A signifies the group

$$\overset{\oplus}{N}X_1X_2X_3,$$

$-(CH_2)_y-$ can also be substituted by a carboxyl group, characterised in that one starts from compounds of formula II according to claim 1 and when the radicals $R_1$, $R_2$ and $R_3$ are saturated or only one of the radicals is unsaturated

a) splits off the trityl group under weakly acidic conditions and then converts the thereby resulting OH group by etherification (for an arylalkyl radical different from benzyl) or acylation into a saturated group $OR_1$ or $OR_3$ or by phosphorylation into a group $OR_3$, thereafter

b) rearranges the allyl group into the propylene group and slits this off and the thereby resulting OH group, if desired, then converted by etherification (for an arylalkyl radical different from benzyl) or acylation into a saturated group $OR_1$, $OR_2$ or $OR_3$ or by phosphorylation into a group $OR_3$, whereby the rearrangement of the allyl group into the propylene group can also already take place in step a) before the etherification, acylation or phosphorylation, and subsequently, if desired

c) removes the benzyl group by catalytic hydrogenolysis and converts the thereby resulting OH group, if desired, by etherification (for an arylalkyl radical different from benzyl) or acylation into a saturated or unsaturated group $OR_1$, $OR_2$ or $OR_3$ or by phosphorylation into a group $OR_3$, or when the radicals are saturated or unsaturated,

$a_1$) splits off the trityl group and etherifies the resultant OH group with a tetrahydropyranyl group, thereafter

$b_1$) splits off the allyl group and converts the resulting OH group by etherification (for an arylalkyl radical different from benzyl) or acylation into a saturated group $OR_1$, $OR_2$ or $OR_3$ or by phosphorylation into a group $OR_3$,

$c_1$) thereafter splits off the tetrahydropyranyl group or, if desired, the benzyl group and converts the resulting OH group by etherification (for an arylalkyl radical different from benzyl) or acylation into a saturated (after splitting off of the tetrahydropyranyl group) or saturated or unsaturated (after splitting off of the benzyl group) group $OR_1$, $OR_2$ or $OR_3$ or by phosphorylation into a group $OR_3$ and subsequently

$d_1$) splits off the remaining tetrahydropyranyl group or, if desired, the benzyl group and, if desired, converts the resulting OH group by etherification (for an arylalkyl radical different from benzyl) or acylation into a saturated or unsaturated group $OR_1$, $OR_2$ or $OR_3$ or by phosphorylation into a group $OR_3$, or

$a_2$) splits off the trityl group and acylates the resulting OH group with a long-chained saturated fatty acid with 10 to 25 C-atoms, thereafter

$b_2$) splits off the allyl group and etherifies the resulting OH group with a tetrahydropyranyl group and

$c_2$) if desired, splits off the long-chained acyl radical and converts the resulting OH group by etherification (for an arylalkyl radical different from benzyl) or acylation into a saturated group $OR_1$ or $OR_3$ or by phosphorylation into a group $OR_3$, and thereafter

$d_2$) splits off the tetrahydropyranyl group or, if desired, the benzyl group and converts the resulting OH group by etherification (for an arylalkyl radical different from benzyl) or acylation into a saturated (after splitting off of the tetrahydropyranyl group) or saturated or unsaturated (after splitting off of the benzyl group) group $OR_1$, $OR_2$ or $OR_3$ or by phosphorylation into a group $OR_3$ and subsequently

$e_2$) splits off the remaining tetrahydropyranyl group or, if desired, the benzyl group and, if desired, converts the resulting OH group by etherification (for an arylalkyl radical different from benzyl) or acylation into a saturated or unsaturated group $OR_1$, $OR_2$ or $OR_3$ or by phosphorylation into a group $OR_3$, and, if desired, reacts a compound obtained of the formula I, in which A is a halogen atom, with a compound

$$\overset{\oplus}{N}X_1X_2X_3$$

or converts a compound obtained of the formula I, in which A is an

$$\overset{\oplus}{N}H_3$$

group, into a compound of formula I, in which A is an

$$\overset{\oplus}{N}X_1X_2X_3$$

group.

6. Process according to claim 5, characterised in that the alkyl radical R in the radical —COR has 1 to 3 C-atoms.

7. Process according to claim 5, characterised in that the alkyl radical R in the radical —COR has 9 to 19 C-atoms.

8. Process according to claim 5 or 7, characterised in that the acyl group of the radical R is of a natural fatty acid.

9. Process according to one of claims 5 to 8, characterised in that one starts from 1-trityl-2-allyl-3-benzyl-sn-glycerol.

10. Process according to one of claims 5 to 8, characterised in that one starts from 1-trityl-2-benzyl-3-allyl-sn-glycerol.

**Claims for the Contracting State: AT**

1. Process for the preparation of glycerol derivatives of the formula II:

$$\begin{array}{l} CH_2—O—R'_1 \\ | \\ CH—O—R'_2 \\ | \\ CH_2—O—R'_3 \end{array} \qquad (II)$$

wherein each of the radicals $R'_1$, $R'_2$ and $R'_3$ is different and signifies a benzyl group or allyl group, $R'_1$ and $R'_3$ also a trityl group, in that into a 1- (or 3-) benzyl- (or allyl-) glycerol one introduces the trityl group into the 3- (or 1-) position and thereafter the allyl (or benzyl) group into the 2-position.

2. Process for the further working up of the compounds of formula II prepared according to the process of claim 1 for the preparation of glycerol derivatives of formula I:

$$\begin{array}{l} CH_2—O—R_1 \\ | \\ CH—O—R_2 \\ | \\ CH_2—O—R_3 \end{array} \qquad (I)$$

wherein each of the radicals $R_1$, $R_2$ and $R_3$ is different and represents a saturated alkyl radical R, a saturated acyl radical —COR or hydrogen, whereby R signifies a straight-chained or branched alkyl group with 1 to 25 C-atoms or an arylalkyl radical with 1 to 25 C-atoms in the alkyl chain and wherein two of the radicals can also be unsaturated and $R_3$ can also signify the group

$$\begin{array}{l} —P(O)—O—(CH_2)_y—A \\ \quad | \\ \quad O^{\ominus}(Z^{\oplus})_n \end{array}$$

wherein A is a halogen atom, the group

$$-\overset{\oplus}{N}X_1X_2X_3$$

or also hydrogen, $X_1$, $X_2$ and $X_3$ are the same or different and signify hydrogen or a straight-chained or branched alkyl group with 1 to 6 C-atoms, y is a whole number from 2 to 12 or, when A is a hydrogen atom, can also be 0, $Z^{\oplus}$ is an equivalent of a mono- to trivalent cation and n is 1 or, when A signifies the group

$$\overset{\oplus}{N}X_1X_2X_3$$

is 0 and wherein, when A signifies the group

$$\overset{\oplus}{N}X_1X_2X_3,$$

$-(CH_2)_y-$ can also be substituted by a carboxyl group, characterised in that one starts from compounds of formula II according to claim 1 and when the radicals $R_1$, $R_2$ and $R_3$ are saturated or only one of the radicals is unsaturated

a) splits off the trityl group under weakly acidic conditions and then converts the thereby resulting OH group by etherification (for an arylalkyl radical different from benzyl) or acylation into a saturated group $OR_1$ or $OR_3$ or by phosphorylation into a group $OR_3$, thereafter

b) rearranges the allyl group into the propylene group and splits this off and the thereby resulting OH group, if desired, then converted by etherification (for an arylalkyl radical different from benzyl) or acylation into a saturated group $OR_1$, $OR_2$ or $OR_3$ or by phosphorylation into a group $OR_3$, whereby the rearrangement of the allyl group into the propylene group can also already take place in step a) before the etherification, acylation or phosphorylation, and subsequently, if desired

c) removes the benzyl group by catalytic hydrogenolysis and converts the thereby resulting OH group, if desired, by etherification (for an arylalkyl radical different from benzyl) or acylation into a saturated or unsaturated group $OR_1$, $OR_2$ or $OR_3$ or by phosphorylation into a group $OR_3$, or when the radicals are saturated or unsaturated,

$a_1$) splits off the trityl group and etherifies the resultant OH group with a tetrahydropyranyl group, thereafter

$b_1$) splits off the allyl group and converts the resulting OH group by etherification (for an arylalkyl radical different from benzyl) or acylation into a saturated group $OR_1$, $OR_2$ or $OR_3$ or by phosphorylation into a group $OR_3$,

$c_1$) thereafter splits off the tetrahydropyranyl group or, if desired, the benzyl group and converts the resulting OH group by etherification (for an arylalkyl radical different from benzyl) or acylation into a saturated (after splitting off of the tetrahydropyranyl group) or saturated or unsaturated (after splitting off of the benzyl group) group $OR_1$, $OR_2$ or $OR_3$ or by phosphorylation into a group $OR_3$ and subsequently

$d_1$) splits off the remaining tetrahydropyranyl group or, if desired, the benzyl group and, if desired, converts the resulting OH group by etherification (for an arylalkyl radical different from benzyl) or acylation into a saturated or unsaturated group $OR_1$, $OR_2$ or $OR_3$ or by phosphorylation into a group $OR_3$, or

$a_2$) splits off the trityl group and acylates the resulting OH group with a long-chained saturated fatty acid with 10 to 25 C-atoms, thereafter

$b_2$) splits off the allyl group and etherifies the resulting OH group with a tetrahydropyranyl group and

$c_2$) if desired, splits off the long-chained acyl radical and converts the resulting OH group by etherification (for an arylalkyl radical different from benzyl) or acylation into a saturated group $OR_1$ or $OR_3$ or by phosphorylation into a group $OR_3$, and thereafter

$d_2$) splits off the tetrahydropyranyl group or, if desired, the benzyl group and converts the resulting OH group by etherification (for an arylalkyl radical different from benzyl) or acylation into a saturated (after splitting off of the tetrahydropyranyl group) or saturated or unsaturated (after splitting off of the benzyl group) group $OR_1$, $OR_2$ or $OR_3$ or by phosphorylation into a group $OR_3$ and subsequently

$e_2$) splits off the remaining tetrahydropyranyl group or, if desired, the benzyl group and, if desired, converts the resulting OH group by etherification (for an arylalkyl radical different from benzyl) or acylation into a saturated or unsaturated group $OR_1$, $OR_2$ or $OR_3$ or by phosphorylation into a group $OR_3$, and, if desired, reacts a compound obtained of the formula I, in which A is a halogen atom, with a compound

$$\overset{\oplus}{N}X_1X_2X_3$$

or converts a compound obtained of the formula I, in which A is an

$$\overset{\oplus}{N}H_3$$

group, into a compound of formula I, in which A is an

$$\overset{\oplus}{N}X_1X_2X_3$$

group.

3. Process according to claim 2, characterised in that the alkyl radical R in the radical —COR has 1 to 3 C-atoms.

4. Process according to claim 1, characterised in that the alkyl radical R in the radical —COR has 9 to 19 C-atoms.

5. Process according to claim 2 or 4, characterised in that the acyl group of the radical R is of a natural fatty acid.

6. Process according to one of claims 1 to 5, characterised in that one starts from 1-trityl-2-allyl-3-benzyl-sn-glycerol.

7. Process according to one of claims 2 to 5, characterised in that one starts from 1-trityl-2-benzyl-3-allyl-sn-glycerol.